# EUROPEAN PATENT SPECIFICATION

(11) **EP 1 187 588 B1**
(45) Date of publication and mention of the grant of the patent: **02.08.2006**
(21) Application number: 00931954.2
(22) Date of filing: 27.04.2000
(51) Int. Cl.: A61F 13/15, B32B 27/00, D06M 13/244, D06M 15/01, D06M 15/333

(54) **ABSORBENT ARTICLE COMPRISING TOPSHEET WITH LOW SURFACTANT OR NO SYNTHETIC SURFACTANT**
ABSORBIERENDER ARTIKEL MIT OBERER SCHICHT WELCHE EIN NIEDRIGES TENSID ODER KEIN SYNTHETISCHES TENSID ENTHÄLT
ARTICLE ABSORBANT COMPRENANT UNE COUCHE SUPERIEURE A FAIBLE TENEUR DE TENSIOACTIF OU SANS TENSIOACTIF SYNTHETIQUE

(30) Priority: 29.04.1999 US 301744
(43) Date of publication of application: 20.03.2002
(73) Proprietor: Paragon Trade Brands, Inc., Norcross, Georgia 30092 (US)
(72) Inventor: CHMIELEWSKI, Harry, J., Brunswick, GA 31523 (US); ERDMAN, Carol, L., Duluth, GA 30095 (US)
(74) Representative: Meddle, Alan L.
(86) International application number: PCT/US2000/011185
(87) International publication number: WO 2000/066058

(56) References cited:
- EP-A- 0 598 204
- EP-A- 1 051 959
- WO-A-96/10381
- WO-A-97/31153
- WO-A-98/24618
- WO-A-99/32706
- US-A- 5 057 361
- US-A- 5 620 788
- US-A- 5 944 705

## Description

### FIELD OF THE INVENTION

This invention pertains to an absorbent article, such as a disposable diaper, sanitary napkin, adult incontinence garment, training pant or the like, which at a minimum comprises a topsheet, and particularly a topsheet that has been treated to render the topsheet hydrophilic with minimal levels of surfactants.

### BACKGROUND OF THE INVENTION

Commonly, an absorbent article, such as a disposable diaper, adult incontinent garment, training pant or sanitary napkin, comprises a topsheet which is at least partially liquid permeable, a liquid-impermeable backsheet, and an absorbent core formed from (1) cellulosic fibers, which typically are comminuted softwood pulp fibers, and (2) distributed particles of a superabsorbent polymer (SAP). The absorbent core is generally positioned between the topsheet and the backsheet. It is known to provide the absorbent article with one or more other layers formed from cellulosic fibers or other materials to perform various liquid-absorbing, liquid-distributing, and cushioning functions.

Polymeric materials are generally used to form the topsheet of such absorbent articles. Polyolefin nonwovens are particularly suited for making these type of products. Absorbent articles desirably include a polymeric nonwoven topsheet which provides rapid intake of fluids such as water or urine, minimal spreading of fluid on the surface before fluid penetration, and good wettability. The topsheet should be durable enough to survive multiple fluid insults.

Polyolefin nonwovens and other types of polymeric fabrics are normally hydrophobic. In order to effectively absorb water, the polymeric fabrics must be treated to become hydrophilic. Polymeric fabrics may be rendered hydrophilic by applying a surfactant to the fabric.

Conventional surfactants for treating polymeric fabrics include non-ionic surfactants such as octylphenoxypolyethoxy ethanol. Although such conventional surface treatments are effective in making polymeric fabrics wettable, there are still some problems. For example, conventional surface treatment compositions are relatively easily rubbed-off the fabric, particularly when the fabric is wetted. Such surface treatments are often substantially completely removed from the polymeric fabric after only one liquid insult or washing. After the surface treatment is removed, the polymeric fabric again becomes water-repellent and less effective in absorbing water.

In order to compensate for the tendency of conventional surface treatments to rub off, conventional surface treatments are often applied to the polymeric fabrics in large quantities. Heavy applications lead to increased costs. Further, such levels of surfactants have been known to cause skin irritation in some individuals, particularly those with sensitive skin. Generally, surfactant levels as high as 1% by weight of the treated portion of the topsheet, and more specifically between 0.3% and 0.6% by weight of the treated portion of the topsheet, have been used. In the past, it was generally believed that applications any less than these would not permit adequate wetting of the topsheet.

For example, U.S. Patent No. 5,057,361 to Sayovitz et al. discloses a durable surface treatment for improving the wettability of polymeric fabrics, and particularly treatment of polymeric fabrics with a primary surfactant having a low solubility in water. The primary surfactant is applied to the fabric in an aqueous solution along with a fugitive co-surfactant or co-wetting aid which wets the polymeric fabric during application of the primary surfactant and provides for substantially uniform distribution of the primary surfactant onto the fabric. Although the surface treatment disclosed in the Sayovitz '361 Patent is said to improve wettability of polymeric fabrics, there is still a risk of skin irritation associated with the relatively high levels of surfactants used.

Likewise, U.S. Patent No. 5,620,788 to Garavaglia et al. discloses a wettable polymeric fabric comprising a normally water repelling fabric and a sorbitol succinate surfactant substantially uniformly distributed or the surface of the fabric. Suitable succinate surfactants are said to include ethoxylated amino sorbitol succinate salt and alkenyl succinate anhydride ethoxylated fatty amine salt. The succinate surfactant is substantially uniformly distributed on the surface of the polymeric fabric in an aqueous composition comprising a co-wetting aid. The co-wetting aid reduces the surface tension of the aqueous composition and is present in an amount sufficient so that the surfactant is uniformly distributed on the surface of the polymeric fabric during application of the aqueous composition to the surface of the polymeric fabric.

The '788 Patent to Garavaglia also discloses that the polymeric fabric includes the first surfactant in an effective amount up to about 3% by weight of the treated portion of the fabric and the second surfactant in an effective amount up to about 3% by weight of the treated portion of the fabric. Preferably, the polymeric fabric includes the first surfactant in an amount from about 0.1% to about 3.0% by weight of the fabric and the second surfactant in an amount from about 0.1% to about 3.0% by weight of the fabric. Although the polymeric fabric disclosed in the '788 Patent is said to improve wettability, the levels of surfactant used still have the potential to cause skin irritation.

WO99/32706 describes a composition for treating nonwovens. The composition includes a surfactant and a skin wellness additive including aloe vera, and may be applied to nonwovens as an aqueous emulsion.

WO96/10381 describes an absorbent article comprising a nonwoven having a surface treated by an aqueous dispersion of a surfactant and polyvinyl alcohol.

Among other things, this invention has resulted from ongoing efforts to improve the wettability of the topsheet of an absorbent article, while minimizing the use of surfactants which may cause skin irritation without sacrificing topsheet performance.

### SUMMARY OF THE INVENTION

Accordingly, an object of the present invention is to provide an absorbent article having an improved treated topsheet.

Another object of the present invention is to provide an absorbent article having a treated topsheet with enhanced wettability and which at the same time reduces the potential for skin irritation by employing lower levels of surfactants than have been used in the past.

Accordingly, in a first aspect, the present invention provides an absorbent article comprising a nonwoven of hydrophobic fibers, a backsheet and an absorbent core disposed between said nonwoven and said backsheet, wherein at least one target surface of said nonwoven is treated with an aqueous solution comprising a surfactant and a hydrophilic polymer, and said aqueous solution is dried to form a coating, characterised in that said coating is present in an amount ranging from 0.10% to 0.25% by weight of the target surface, said coating comprises from 10% to 50% by weight of water-soluble hydrophilic polymer and from 50% to 90% by weight of said surfactant, and said absorbent article further comprises a topsheet, said nonwoven serving as a transfer layer positioned between said absorbent core and said topsheet.

In a second aspect, the present invention provides a method for treating a nonwoven for use in an absorbent article, the method comprising the steps of: preparing an aqueous solution comprising a surfactant and a water-soluble hydrophilic polymer; applying said aqueous solution to a target surface of the nonwoven; and drying the nonwoven to form a coating on said target surface of the nonwoven, characterised in that: said aqueous solution comprises said hydrophilic polymer and said surfactant at a ratio of 30:70, whereby the hydrophilic polymer and surfactant coat said target surface on drying of the nonwoven; the hydrophilic polymer and the surfactant are present in the aqueous solution at a total concentration ranging from 5% to 25% by weight of the total aqueous solution; and the surface tension of said aqueous solution is less than 40 mN/m.

In a preferred embodiment, the present invention is directed to a topsheet for an absorbent garment. The topsheet is treated with less surfactant than previously used in prior art absorbent garments. Reduced surfactant levels are believed to provide a topsheet that would be potentially milder to sensitive skin. This invention is in part premised on the unexpected discovery that certain combinations of surfactants and hydrophilic polymers provide equal or improved topsheet performance while using a fraction of the surfactant which has conventionally been employed. The surfactant and hydrophilic polymers are mixed in an aqueous solution and applied to the topsheet in amounts sufficient to promote good wettability for urine absorption.

These and other objects, features and advantages of this invention are evident from the following description of the preferred embodiments of this invention, with reference to the accompanying drawings.

### BRIEF DESCRIPTION OF THE DRAWINGS

FIG. 1 is a fragmentary, perspective view of a disposable diaper exemplifying an absorbent article according to this invention, in an assembled condition.
FIG. 2 is a fragmentary, perspective view of the disposable diaper of FIG. 1, in a flattened condition.

### DETAILED DESCRIPTION OF THE PREFERRED EMBODIMENTS

The present invention is directed to an absorbent article such as a diaper, training pants, adult incontinent products and sanitary napkins, comprising a topsheet, wherein at least a portion of the topsheet has been treated with an aqueous solution comprising a hydrophilic polymer and optionally an effective amount of a surfactant.

Though the present invention is disclosed primarily in reference to surface treatments of nonwovens used as topsheets, it will be readily apparent to those skilled in the art that the low surfactant treatments can be applied to non-woven structures used as transfer or acquisition sublayers of absorbent garments. In this case, the non-woven usually comprises polyester, polypropylene or polyethylene fibers.

The surface treated topsheet of the preferred embodiments is preferably formed from polymeric fabrics such as polyolefin nonwoven fabrics. Common polyolefin nonwoven fabrics include polypropylene and polyethylene spunbonded fabrics. Such fabrics are typically produced by processes disclosed in the following patents: U.S. Patent No. 4,340,563 to Appel et al.; U.S. Patent No. 3,692,618 to Dorschner et al.; U.S. Patent No. 3,338,992 to Kinney; U.S. Patent No. 3,341,394 to Kinney; U.S. Patent No. 3,502,538 to Levy; U.S. Patent No. 3,502,763 to Hartmann; U.S. Patent No. 3,909,009 to Hartmann; and U.S. Patent No. 3,542,615 to Dobo et al.. Additionally, the topsheet of the present invention can be formed from nonwoven bicomponent polymeric fabrics. Nonwoven bicomponent fabrics are typically produced by processes such as are disclosed in U.S. Patent No. 3,423,266 to Davies et al. and U.S. Patent No. 3,595,731 to Davies, et al..

The wettability of the normally hydrophobic topsheet is enhanced according to the present invention by the application of an aqueous solution comprising a hydrophilic polymer to at least a portion of the topsheet. A hydrophilic film is thereby formed on the surface of selected portions of the topsheet to render it hydrophilic without the addition of elevated levels of surfactants, which have the potential to cause skin irritation to sensitive or damaged skin, i.e., skin already exhibiting diaper rash. Different surfactants are traditionally applied at different levels. By way of example, but without intending to limit the invention, dioctyl sodium sulfosuccinate sold as TRITON ^{™} GR-5M (manufactured by Union Carbide of Danbury, CT) is typically applied to spunbonded nonwoven so that, when dry, it comprises about 0.3% by weight of the treated portion of the topsheet. Alternatively, sodium alkylarylpolyethersulfonate sold as TRITON^{™} X-200 may be applied to the topsheet. Another surfactant, SILASTOL^{™} PST (manufactured by Schill & Seilacher of Böblingen, Germany), is recommended to be applied at a rate so that, when dry, it comprises about 0.4-0.5% of the treated portion of the spunbond nonwoven topsheet. Treatments above these levels are believed to provide the potential to cause skin irritation.

To render a nonwoven hydrophilic, it is necessary to coat the surfaces of individual fibers which form the topsheet. Accordingly, the surface tension of the aqueous solution must be sufficiently low in order to wet the hydrophobic nonwoven during application of the aqueous coating and subsequent drying.

In a preferred embodiment of the present invention, the aqueous solution comprising the hydrophilic polymer is applied to at least one portion of the topsheet in conjunction with low, non-irritating levels of a surfactant. According to the present invention, it has been unexpectedly discovered that surfactant may be used in quantities much lower than those previously used to treat topsheets, without sacrificing topsheet performance. The surfactant facilitates the application of the aqueous solution to the surface of the topsheet by lowering the surface tension of the aqueous solution containing the hydrophilic polymer. In other words, the surfactant enhances the ability of the hydrophilic polymer to coat the surface of the treated portions of the topsheet.

The hydrophilic polymer of the present invention is preferably one suitable for use in hygienic applications and which can substantially coat the surface of the fibers of the treated portions of the topsheet to render those portions of the topsheet hydrophilic. The hydrophilic polymer is preferably applied with or without the aid of a surfactant. Preferably, the hydrophilic polymer is a non-surface active polymer such as aloe vera, methylcellulose, hydroxypropyl methylcellulose, gum arabic, gum acacia, and polyvinyl alcohol.

The hydrophilic polymer is present in the aqueous solution in an amount effective to substantially coat the treated portion of the topsheet, with lower than conventional applications of surfactant, i.e., 0.25% when dry. The term "substantially coat" means that the fibers forming the topsheet are substantially entirely coated with the hydrophilic polymer to such a degree as to render the surface of the topsheet hydrophilic. Preferably, the hydrophilic polymer, when dry, is present on the treated portion of the topsheet in an amount at least about 0.05%-1.0% by weight of the treated portion of the topsheet, with a particularly preferred range being about 0.05% to about 0.1% by weight of the dry treated portion of the topsheet. Given the expense of the hydrophilic polymer, particularly aloe, the upper limit of polymer application is primarily defined by economic considerations, though higher levels of the hydrophilic polymer are contemplated by the present invention, such as where the hydrophilic polymer is less expensive, e.g., where methylcellulose or gum arabic are used.

If employed, the optional surfactant can be any known surfactant suitable for use in skin contact hygienic applications, as is generally known in the art, and should have a surface tension which is less than the apparent surface free energy of the topsheet. For example, an untreated polypropylene nonwoven fabric, which is hydrophobic, typically has an apparent surface free energy of about 36 ergs/cm². Polyester nonwoven, used as a transfer layer, has a surface free energy of about 43 ergs/cm² . Particularly preferred surfactants for use in the present invention include, but are not limited to, TRITON^{™} GR-5M and SILASTOL^{™} PST.

In the preferred embodiments, it is desirable, though not required, to employ the lowest amount of surfactant which is effective in spreading the hydrophilic polymer on the surface of the treated portion of the topsheet. This invention is premised in part on the unexpected discovery that hydrophobic topsheets may be rendered hydrophilic with a fraction of the surfactant than has been employed in the past, when combined with certain hydrophilic polymers, all the while sustaining and even in some cases improving topsheet performance. The surfactant is present in the aqueous solution sufficient to lower the surface tension of the aqueous solution to a level below about 40 mN/m, more preferably below about 35 mN/m, and most preferably below about 32 mN/m. At these surface tensions, the aqueous solution effectively wets the untreated portion of the topsheet and coats that portion with the hydrophilic polymer.

By way of example, but without limiting the invention, the surface tension of water is about 72.8 mN/m, and an aloe vera/water solution has a surface tension of about 71 mN/m. At the preferred concentrations, for example about 30:70 ratio of aloe vera to surfactant at a total solids concentration of about 1%, the surface tension of an aloe vera/surfactant water solution is about 31 mN/m. A sufficient amount of the aqueous solution is applied to the nonwoven such that upon drying the surfactant is present on the treated portions of the topsheet so that when dry it comprises less than about 0.25% by weight of the treated portions of the topsheet, more preferably less than about 0.125% by weight of the treated portions of the topsheet, and most preferably less than about 0.1 % by weight of the treated portions of the topsheet.

There is evidence that the combination of the surfactant in combination with the hydrophilic polymer provides an otherwise unexpected synergistic result. A 0.82% concentration SILASTOL^{™} PST treatment solution has a surface tension of about 35.3 mN/m. By comparison, a 0.25% concentration of aloe vera in a treatment solution has a surface tension of about 71.4 mN/m. Surprisingly, a solution consisting of 0.82% PST and 0.25% aloe vera has a surface tension of about 31.5 mN/m. In other words, the addition of aloe vera at a 0.25% concentration to a 0.82% SILASTOL^{™} PST solution lowers the surface tension from 35.3 mN/m to 31.5 mN/m. It is surprising to discover that a non-surface active polymer, such as aloe vera, can further reduce the surface tension of a surfactant solution by an additional ten percent.

The aqueous solution comprising the aloe vera and surfactant is prepared by vigorously pre-mixing the required amount of aloe vera with a portion, e.g., about 10%, of the total water needed for the batch application. This premix is added to the remainder of the water. The surfactant is then added to the solution. This solution may be prepared at room temperature. This method of preparation is applicable to spray and kiss-coat (also known as etched roll or gravure roll) applications, but not foam applications. Foam applications are generally prepared on-line. In this case, each of the surfactant and hydrophilic polymer are supplied from master tanks at the appropriate concentrations to a foam generator as is known to those skilled in the art.

The target dry add-on concentration can be achieved by adjusting the concentration of total solids in the aqueous solution and the amount of aqueous solution applied to the nonwoven. Those skilled in the art will recognize many ways to arrive at the preferred target dry concentration by appropriate modification of the foregoing preferred levels for specific methods of application.

Low levels of hydrophilic additives such as chamomile and echinacea, and low levels of hydrophobic additives such as Vitamin E, tocopherol acetate, and hamamelis can be added at levels up to less than about 10% of the hydrophilic polymer without significantly reducing the performance of the topsheet. These additives are recognized as providing potential benefits in skin contact hygienic applications.

In another preferred embodiment, the synthetic surfactant may be replaced with phospholipids such as phosphoglycerides and sphingolipids. Particularly preferred phosphoglycerides are lecithin and cephalins. Lecithin is a naturally occurring surfactant. When lecithin is employed, no amount of additional surfactant is required to enhance the ability of the hydrophilic polymer to coat the surface of the treated portions of the topsheet.

After the nonwoven is wetted with the aqueous solution, it is preferably dried by, e.g., directing the nonwoven through a forced hot air oven or across a bank of infrared lights, steam cans, dielectric dryers or other conventional drying apparatuses as are known to those skilled in the art. The non-woven typically moves across the heating medium at the line speed at which the aqueous solution is applied. Typical line speeds for application of the aqueous solution are around 2.54-5.08 cm/s (500-1000 ft/min).

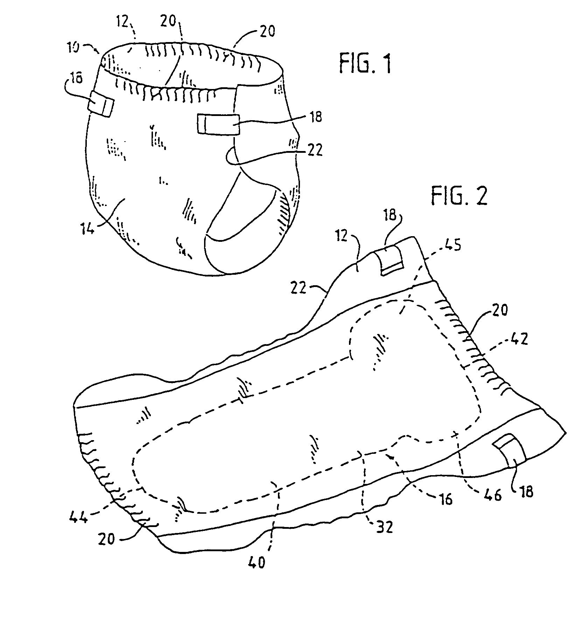

As shown in FIGS. 1 and 2, the topsheet treated with the aqueous solution may be employed, for example, in a disposable diaper 10. The disposable diaper 10 may be appropriately sized for infant use or for adult use. If sized for adult use, the disposable diaper 10 may be also called an incontinent garment. It may be here noted that this invention may be also embodied in a wound dressing or another absorbent article other than a disposable diaper, e.g., training pant, adult incontinence garment, sanitary napkin, or the like.

Broadly, the disposable diaper 10 comprises a topsheet 12 as described above, a liquid-impermeable backsheet 14, and an absorbent core 16 positioned between the topsheet 12 and the backsheet 14. The disposable diaper 10 has tape fasteners 18, elasticized waistbands 20, and other features well known to those skilled in the art. The topsheet 12 and the backsheet 14 may be bonded adhesively around outer edges 22 of the disposable diaper 10, in a known manner, so as to encapsulate the absorbent structure 16. As described above, the topsheet 12, also called a facing sheet, may be made from polymeric fibers such as polyolefins. The backsheet 14 may be made from a synthetic polymeric film, such as a polyethylene film.

The disposable diaper 10 may be substantially similar to the disposable diaper disclosed in Huffman et al. U.S. Patent No. 5,403,301, or in Chmielewski U.S. Pat No. 5,891,120, both assigned to the assignee of the present invention. As shown in FIG. 2, the absorbent core 32 has an elongate, central portion 40 with a front end 42 and a back end 44, along with two ears 46 near the front end 44.

The topsheet 12 is treated with an aqueous solution comprising a hydrophilic polymer according to the present invention to substantially coat the surface of at least a portion of the topsheet i.e. the target surface of the topsheet to thereby render the topsheet hydrophilic. In one embodiment of the present invention, at least about 35% of the surface of the topsheet is treated. Preferably, the treated portion of the topsheet 12 is that portion which generally corresponds in location to the absorbent core 32, and more preferably that portion which corresponds in location to the central portion 40 of the absorbent core 32.

When the topsheet of the preferred embodiments is incorporated into diapers, other diaper configurations are possible without departing from the scope of the preferred embodiments. For instance, in-board leg gathers, i.e., elasticized standing leg gathers disposed inboard of the leg elastics, may be employed. In this situation, the portion of the topsheet located between the standing leg cuffs or gathers is preferably treated with the aqueous solution to render it hydrophilic. The outer portions of the topsheet in this configuration may be left untreated, as urine insults are generally confined between the standing leg cuffs. The untreated outboard portions of the topsheet would advantageously reduce rewetting and contribute to overall liquid containment.

In an alternative embodiment, the nonwoven topsheet could be substituted with an apertured plastic film. In this alternative configuration, the apertured plastic film serving as the topsheet is treated in a manner similar to the nonwoven topsheet described previously. The apertured film topsheet, as treated with the surfactant, is rendered hydrophilic with correspondingly less surfactant than has been used in the prior art. Advantageously, such apertured film topsheets also exhibit performance criteria, such as strikethrough and rewet, which are as good as, or better than, conventional topsheets employing higher concentrations of surfactant.

Surface treatment processes within the scope of the present invention include kiss-coating and spraying methods. Suitable kiss-coating and spraying methods are disclosed in U.S. Patent No. 5,620,788 to Garavaglia et al. and U.S. Patent No. 5,635,191. Other methods include various foam application techniques. However, it should be understood that the practice of the present invention is not limited to the above-described methods.

The following examples are designed to illustrate particular embodiments of the present invention and demonstrate the efficacy of the present invention as compared to conventional surfactant-containing absorbent articles.

### Example 1

### Strikethrough/Rewet of Diapers Made with Low Surfactant Topsheet Treated with TRITON^{™} or SILASTOL^{™}

### Sample Description:

Paragon Trade Brands' Size Large Ultra 3 diapers were made according to the products' specifications, except that the central portions of the topsheet were treated according to the following three spray application concentrations: 1) 0.075% SILASTOL^{™} PST, 0.05% aloe; 2) 0.075% TRITON^{™} GR-5M, 0.05% aloe; and 3) control standard topsheet treated with 0.3% TRITON^{™} GR-5M. All numerical values are percentages by weight of the treated portions of the topsheet.

### Results and Conclusions:

Based on the results in Table 1 below, both surfactants in the low surfactant topsheet formula (treatments (1) and (2) above) of the preferred embodiments perform similar to the control topsheet (treatment (3) above) for strikethrough/rewet testing of diapers. Notably, treatments (1) and (2) employ only a fraction of the surfactant of the control, yet topsheet performance is not compromised.

Strikethrough is a measure of the rate of absorption of the diaper. Rewet is a measure of the surface wetness of the diaper. Both are affected by the wettability of the topsheet. The strikethrough test is performed by laying the diaper flat and placing an EDANA (European Disposables and Nonwovens Association) dosing head on the surface of the diaper topsheet. The prescribed amount of fluid, namely 100 ml. of a solution containing 0.9% saline, is metered into the EDANA dosing head. The time required for all fluid to be absorbed into the diaper core is recorded as the strikethrough time in seconds. After the first dose, the diaper is placed under a load of 0.5 psi for 10 minutes. Then, about 18 grams of pre-weighed dried filter paper is placed on top of the diaper core under a load of 3.4. 10³ Pa (0.5 psi) for 10 minutes. The amount of test fluid absorbed by the filter paper is recorded as the rewet in grams. This test is then repeated for second and third doses. The results obtained on six diapers are averaged and tabulated below.

**TABLE 1 Performance of Low Surfactant Topsheets on Diapers**

| | | **Strikethrough (seconds)** | | | **Rewet (grams)** | | |
|---|---|---|---|---|---|---|---|
| TREATMENT | | **1**^{**st**} | **2**^{**nd**} | **3**^{**rd**} | **1**^{**st**} | **2**^{**nd**} | **3**^{**rd**} |
| Control: | Avg. | 58 | 83 | 88 | 0.4 | 22.9 | 44.7 |
| 0.3% TRITON^{™} | | | | | | | |
| GR-5M | Std. Dev. | 5 | 12 | 30 | 0.2 | 8.0 | 11.5 |
| 0.075% TRITON^{™} | Avg. | 45 | 66 | 86 | 0.3 | 14.0 | 43.5 |
| GR-5M | | | | | | | |
| 0.05% aloe | Std. Dev. | 4 | 2 | 11 | 0.1 | 3.8 | 6.5 |
| 0.075% | Avg. | 44 | 66 | 73 | 0.3 | 19.9 | 38.8 |
| SILASTOL^{™} PST | | | | | | | |
| 0.05% aloe | Std. Dev. | 3 | 13 | 11 | 0.1 | 4.5 | 9.0 |

Based on the hydrophilicity of the low surfactant topsheet treatments, the strikethrough times were comparable or better, i.e., lower, than those obtained for a conventional surfactant treated topsheet (Control). These results reveal the surprising and unexpected discovery that the amount of surfactant employed in rendering a topsheet hydrophilic may be significantly reduced, without sacrificing topsheet performance. Furthermore, it was surprising and unexpected that the low surfactant topsheet provided better, i.e., lower, rewet performance.

Furthermore, as demonstrated above, the amount of surfactant employed in the topsheet treatments of the preferred low surfactant topsheet is substantially less than that of the Control topsheet. In the control topsheet, 0.3% by weight of the treated portion of the topsheet contained surfactant, whereas only 0.075 wt. % of the treated portion of the topsheet contained surfactant in the preferred embodiments, treatments (1) and (2). This represents a 75% reduction in the amount of surfactant employed in the topsheets of the preferred embodiments as compared to the Control topsheet. The hydrophilic polymer in an amount of only 0.05 wt. % is believed to replace the remaining 0.225 wt. % surfactant that was required in the control topsheet. It was an unexpected discovery that in the preferred formulations, the hydrophilic polymer could in such small quantities replace a much larger amount of surfactant, while providing improved topsheet performance.

### Example 2

### Strikethrough/Rewet of Diapers Made with Low Surfactant Topsheet Treated with TRITON^{™} or SILASTOL^{™} and Vitamin E

### Sample Description:

Paragon Trade Brands' Size Large Ultra 3 diapers made in accordance with present product specifications were employed, except that portions of the topsheet were treated according to the following three spray application concentrations: 1) 0.075% SILASTOL™ PST, 0.05% aloe vera and 0.005% Vitamin E, 2) 0.075% TRITON™ GR-5M, 0.05% aloe vera, and 0.005% Vitamin E, and 3) the control standard topsheet treated with 0.3% TRITON^{™} GR-5M (standard Poly Bond topsheet). The topsheets were 0.015 kg/m² (15 gsm) spunbond, made at Poly Bond in Waynesboro, VA.

The strikethrough and rewet testing protocols in this Example 2 are the same as that described above in Example 1.

### Results and Conclusions:

As shown in Tables 2 and 3 below, the performance of the low surfactant topsheets falls within the expected range of results expected for the standard topsheet.

**TABLE 2 Performance of Low Surfactant Topsheets on Diapers**

| | | **Strikethrough (seconds)** | | | **Rewet (grams)** | | |
|---|---|---|---|---|---|---|---|
| **TOPSHEET** | | **1**^{**st**} | **2**^{**nd**} | **3**^{**rd**} | **1**^{**st**} | **2**^{**nd**} | **3**^{**rd**} |
| Control I: | Avg. | 61 | 100 | 116 | 0.7 | 38.9 | 66.9 |
| 0.3% TRITON^{™} | | | | | | | |
| GR-5M | Std. Dev. | 3.9 | 5.2 | 11.3 | 0.7 | 7.8 | 5.8 |
| 0.075% TRITON^{™} | Avg. | 52 | 89 | 95 | 0.3 | 15.9 | 49.8 |
| GR-5M | | | | | | | |
| 0.05% aloe vera | | | | | | | |
| 0.005% Vitamin E | Std. Dev. | 2.1 | 9.5 | 10.5 | 0.0 | 6.4 | 9.6 |
| 0.075% | Avg. | 50 | 82 | 100 | 0.2 | 11.2 | 38.2 |
| SILASTOL^{™} PST | | | | | | | |
| 0.05% aloe vera | | | | | | | |
| 0.005% Vitamin E | Std. Dev. | 6.8 | 4.0 | 11.1 | 0.0 | 7.4 | 12.5 |
| Control II: | | | | | | | |
| 0.075% | Avg. | 53 | 90 | 113 | 0.2 | 11.0 | 45.2 |
| SILASTOL^{™} PST | | | | | | | |
| 0.05% aloe vera | Std. Dev. | 8.8 | 9.8 | 17.7 | 0.1 | 6.7 | 11.4 |

The foregoing results in Table 2 are consistent with the performance of diapers having a low surfactant topsheet in Table 1 above. The addition of hydrophobic materials such as Vitamin E to the low surfactant formulation does not degrade the strikethrough and rewet performance relative to diapers having topsheets with a low surfactant and hydrophilic polymer formulation (Control II).

Another test, multiple void runoff, was also performed on each of the diaper samples of Example 2. Multiple void runoff is a measure of the hydrophilicity of the diaper topsheet. The diaper is stretched flat and inclined at an angle of about 30 degrees. Three 100 ml. doses are applied at a point on the diaper pad that is about two inches from the front end of the diaper core. The doses are applied at a rate of about 7 ml./sec. The amount of fluid running off the end of the diaper is collected and weighed. Under this protocol, the following results of Table 3 were obtained:

**TABLE 3 Multiple Void Runoff Performance of Low Surfactant Topsheets on Diapers**

| **Multiple Void Runoff (grams)** | | | | |
|---|---|---|---|---|
| **TOPSHEET** | | **1**^{**st**} **Total** | **2**^{**nd**} **Total** | **3**^{**rd**} **Total** |
| Control I: | Avg. | 0 | 0 | 4.1 |
| 0.3% TRITON^{™} GR-5M | Std. Dev. | 0 | 0 | 3.6 |
| 0.075% TRITON^{™} GR-5M | Avg. | 0 | 0 | 0 |
| 0.05% aloe vera | | | | |
| 0.005% Vitamin E | Std. Dev. | 0 | 0 | 0 |
| 0.075% SILASTOL^{™} PST | Avg. | 6.3 | 0 | 12.2 |
| 0.05% aloe vera | | | | |
| 0.005% Vitamin E | Std. Dev. | 8.9 | 0 | 3.5 |
| Control II: | | | | |
| 0.075% SILASTOL^{™} PST | Avg. | 0 | 0 | 4.5 |
| 0.05% aloe vera | Std. Dev. | 0 | 0 | 7.8 |

The runoff data of Table 3 demonstrates the unexpected performance advantages of using TRITON^{™} along with aloe vera and Vitamin E over the significantly higher surfactant concentrations of Control I. Even after the third dosing, the TRITON^{™}/aloe/Vitamin E formulation exhibited no runoff, i.e., all of the fluid was collected within the diaper, indicating high topsheet hydrophilicity and high durability of the topsheet surface treatment.

### Example 3

### Strikethrough/Rewet and Multiple Void Runoff of Diapers made with Topsheet Treated with Low Surfactant/Methyl Cellulose

### Sample Description:

Paragon Trade Brands' Size Large diapers were made according to product specifications, except a portion of the topsheet was treated as follows: 0.075% by weight SILASTOL^{™} PST and 0.25% by weight methyl cellulose (i.e., percent by weight of the treated portion of the topsheet). The run speed was 130 fpm. The topsheet was 15 gsm spunbond. Samples were made for testing by removing the topsheet from machine made diapers and replacing the topsheet with the above-described topsheet.

### Results and Conclusions:

As shown in Tables 4 and 5 below, the performance of the low surfactant topsheets coated with methyl cellulose fall within the expected range of results for a standard topsheet and an aloe vera treated topsheet as shown in Tables 1-3 above.

**TABLE 4 Performance of Low Surfactant Topsheets on Diapers**

| | | **Strikethrough (seconds)** | | | **Rewet (grams)** | | |
|---|---|---|---|---|---|---|---|
| **TOPSHEET** | | **1**^{**st**} | **2**^{**nd**} | **3**^{**rd**} | **1**^{**st**} | **2**^{**nd**} | **3**^{**rd**} |
| Topsheet with | Avg. | 30 | 71 | 91 | 3.2 | 17.9 | 49.2 |
| 0.075% | | | | | | | |
| SILASTOL^{™} PST | | | | | | | |
| and | | | | | | | |
| 0.25% methyl | | | | | | | |
| cellulose | Std. Dev. | 7.1 | 21.4 | 18.9 | 6.6 | 5.3 | 6.1 |

**TABLE 5 Multiple Void Runoff Performance of Low Surfactant Topsheets on Diapers**

| **Multiple Void Runoff (grams)** | | | | |
|---|---|---|---|---|
| **TOPSHEET** | | **1**^{**st**} **Total** | **2**^{**nd**} **Total** | **3**^{**rd**} **Total** |
| Topsheet with 0.075% | Avg. | 0 | 0 | 17 |
| SILASTOL^{™} PST and | | | | |
| 0.25% methyl cellulose | Std. Dev. | 0 | 0 | 25 |

### Example 4

### Modified Multiple Void Runoff of Lab Treated Low Surfactant/High Aloe/Vitamin E Topsheet Samples

### Sample Description:

Lab treated samples were prepared using 0.015 kg/m² (15 gsm) untreated spunbond. Samples were cut to approximately 170mm x 480mm. The target treatment was as follows: Sample #1 was treated with an aqueous solution with a target add-on of 0.075% TRITON^{™} GR-5M, 0.05% aloe vera and 0.005% Vitamin E. Sample #2 was treated with an aqueous solution with a target add-on of 0.075% SILASTOL^{™} PST, 0.05% aloe vera and 0.005% Vitamin E. Sample #3 was treated with an aqueous solution with a target add-on of 0.1% SILASTOL^{™} PST, 0.05% aloe vera and 0.005% Vitamin E. The control was a commercial grade topsheet made on a production line, with a target add-on of TRITON^{™} GR-5M of 0.3%.

### Multiple Void Run-off Testing Protocol

This protocol is designed to test the durability and amount of "wash-off" of the surfactant treatment. This test was performed in the same manner as the standard test described above, with the exception that 10 layers of 0.060 kg/m² (60 gsm) airlaid to simulate the diaper core, cut to approximately 200mm x 500mm, were placed under the nonwoven for the absorbent material. The wet airlaid was replaced after each void with 10 layers of dry airlaid.

### Results and Conclusions:

As indicated in Table 6 below, the results for the lab made samples do not indicate any adverse effects from the addition of Vitamin E in a low surfactant treatment. Also, the two surfactants performed similarly for run-off testing in the low surfactant/high aloe/Vitamin E treatment. This data is consistent with that in the Examples above, where a topsheet treated with low concentrations of surfactant were assessed on diapers. As above, the lower concentrations of surfactant, coupled with low concentrations of Vitamin E, did not degrade topsheet performance. It should be noted that failure of a multiple void run-off test requires 20 gms of fluid run-off over three doses. The data obtained for all of the test samples were well within this product specification.

**TABLE 6 Multiple Void Run-Off Performance of Low Surfactant Topsheets**

| **MULTIPLE VOID RUN-OFF (grams)** | | | | |
|---|---|---|---|---|
| **TOPSHEET** | | **1**^{**st**} **dose** | **2**^{**nd**} **dose** | **3**^{**rd**} **dose** |
| Control: (std topsheet) 0.3% TRITON^{™} | Avg: | 0.00 | 0.00 | 0.00 |
| GR-5M | | | | |
| Sample # 1: | | | | |
| 0.075% TRITON^{™} GR-5M, 0.05% aloe | Avg: | 0.00 | 0.00 | 0.00 |
| vera, 0.005% Vitamin E | | | | |
| Sample #2: | | | | |
| 0.075% SILASTOL^{™} PST, 0.05% aloe | Avg: | 0.00 | 0.00 | 0.00 |
| vera, 0.005% Vitamin E | | | | |
| Sample # 3 | | | | |
| 0.1% SILASTOL^{™} PST, 0.05% aloe | Avg: | 0.00 | 0.06 | 0.10 |
| vera, | | | | |
| 0.005% Vitamin E | | | | |

### Example 5

### Multiple Void Runoff Test of Topsheet Made with Low Surfactant/Aloe Vera/Vitamin E

### Sample Description :

A Poly Bond topsheet was treated with an aqueous solution comprising aloe vera and surfactant to obtain a treated topsheet containing 0.075 % by weight surfactant, 0.05 % by weight aloe vera, and 0.01 % by weight Vitamin E. The topsheet was commercially manufactured, 0.016 kg/m² (16 gsm) SMS (spunbond-meltblown-spunbond), untreated, and cut to a sample size of 162mm x 508mm.

### Results and Conclusions:

There was no runoff for the multiple void runoff test protocol described previously. The treatment of the test nonwoven did not wash off alter 3 doses.

The foregoing treated topsheets of the preferred embodiments may be further treated with lotions or other emollients as is well known in the art. Such lotions tend to render the topsheet hydrophobic. The invention of the preferred embodiments provides improved performance and as such provides the opportunity to use lotions and emollients without sacrificing topsheet performance.

Although the invention has been described in connection with the preferred embodiments, these embodiments are not intended to limit the invention. Those skilled in the art will readily appreciate that various modifications may be made to the preferred embodiments.

## Claims (Claims for the following Contracting State(s): FR, DE, GB)

1. An absorbent article comprising a nonwoven of hydrophobic fibers, a backsheet and an absorbent core disposed between said nonwoven and said backsheet,
wherein at least one target surface of said nonwoven is treated with an aqueous solution comprising a surfactant and a hydrophilic polymer, and said aqueous solution is dried to form a coating;
**characterised in that**:
said coating is present in an amount ranging from 0.10% to 0.25% by weight of the target surface;
said coating comprises from 10% to 50% by weight of water-soluble hydrophilic polymer and from 50% to 90% by weight of said surfactant; and
said absorbent article further comprises a topsheet, said nonwoven serving as a transfer layer positioned between said absorbent core and said topsheet.

2. The absorbent article of claim 1, wherein said nonwoven comprises polyolefinic fibers or film.

3. The absorbent article of any preceding claim, wherein said hydrophilic polymer is selected from the group consisting of aloe vera, methylcellulose, hydroxypropyl methylcellulose, gum arabic, gum acacia, and polyvinyl alcohol.

4. The absorbent article of claim 3, wherein said hydrophilic polymer is aloe vera.

5. The absorbent article of any preceding claim, wherein the surfactant is selected from a group consisting of nonionic and anionic surfactants.

6. The absorbent article of claim 5, wherein the surfactant is selected from the group consisting of dioctyl sodium sulfosuccinate (TRITON^{™}GR-5M) and sodium alkylarylpolytethersulfonate (TRITON^{™}X-200).

7. The absorbent article of claim 5, wherein the surfactant is selected from a group of phospholipids known as phosphoglycerides and sphingolipids.

8. The absorbent article of claim 7, wherein said surfactant is selected from the phosphoglycerides group consisting of lecithins and cephalins.

9. The absorbent article of any preceding claim, wherein said surfactant is present on the target surface in an amount less than 0.125% by weight of the target surface.

10. The absorbent article of any preceding claim, wherein said hydrophilic polymer is present on the target surface in an amount ranging from 0.05% to 1 % by weight of the target surface.

11. The absorbent article of claim 10, wherein said hydrophilic polymer is present on the target surface in an amount of 0.05% by weight of the target surface.

12. A method for treating a nonwoven for use in an absorbent article as defined in claim 1 comprising the steps of:
preparing an aqueous solution comprising a surfactant and a water-soluble hydrophilic polymer;
applying said aqueous solution to a target surface of the nonwoven; and
drying the nonwoven to form a coating on said target surface of the nonwoven;
**characterised in that**:
said aqueous solution comprises said hydrophilic polymer and said surfactant at a ratio of 30:70, whereby the hydrophilic polymer and surfactant coat said target surface on drying of the nonwoven;
the hydrophilic polymer and the surfactant are present in the aqueous solution at a total concentration ranging from 5% to 25% by weight of the total aqueous solution; and
the surface tension of said aqueous solution is less than 40 mN/m.

13. The method according to claim 12, wherein said nonwoven is employed as a topsheet in the absorbent article.

## Claims (Claims for the following Contracting State(s): AT, BE, CH, CY, DK, ES, FI, GR, IE, IT, LI, LU, MC, NL, PT, SE)

1. An absorbent article comprising a nonwoven of hydrophobic fibers,
wherein at least one target surface of said nonwoven is treated with an aqueous solution comprising a surfactant and a hydrophilic polymer, and said aqueous solution is dried to form a coating;
**characterised in that**:
said coating is present in an amount ranging from 0.10% to 0.25% by weight of the target surface; and
said coating comprises from 10% to 50% by weight of water-soluble hydrophilic polymer and from 50% to 90% by weight of said surfactant.

2. The absorbent article of claim 1, further comprising a topsheet, a backsheet and an absorbent core disposed therebetween, said nonwoven serving as a transfer layer positioned between said absorbent core and said topsheet.

3. The absorbent article of claim 1, wherein said nonwoven is employed as a topsheet of the absorbent article for contact with the skin.

4. The absorbent article of claim 2 or 3, wherein said nonwoven comprises polyolefinic fibers or film.

5. The absorbent article of any preceding claim, wherein said hydrophilic polymer is selected from the group consisting of aloe vera, methylcellulose, hydroxypropyl methylcellulose, gum arabic, gum acacia, and polyvinyl alchol.

6. The absorbent article of claim 5, wherein said hydrophilic polymer is aloe vera.

7. The absorbent article of any preceding claim, wherein the surfactant is selected from a group consisting of nonionic and anionic surfactants,

8. The absorbent article of claim 7, wherein the surfactant is selected from the group consisting of dioctyl sodium sulfosuccinate (TRITON^{™}GR-5M) and sodium alkylarylpolytethersulfonate (TRITON™X-200).

9. The absorbent article of claim 7, wherein the surfactant is selected from a group of phospholipids known as phosphoglycerides and sphingolipids.

10. The absorbent article of claim 9, wherein said surfactant is selected from the phosphoglycerides group consisting of lecithins and cephalins.

11. The absorbent article of any preceding claim, wherein said surfactant is present on the target surface in an amount less than 0.125% by weight of the target surface.

12. The absorbent article of any preceding claim, wherein said hydrophilic polymer is present on the target surface in an amount ranging from 0.05% to 1% by weight of the target surface.

13. The absorbent article of claim 12, wherein said hydrophilic polymer is present on the target surface in an amount of 0.05% by weight of the target surface.

14. The absorbent article of claim 3, wherein said topsheet comprises a matrix of discrete fibers and the coating is deposited on the fibers of the target surface.

15. A method for treating a nonwoven for use in an absorbent article as defined in claim 1 comprising the steps of:
preparing an aqueous solution comprising a surfactant and a water-soluble hydrophilic polymer;
applying said aqueous solution to a target surface of the nonwoven; and
drying the nonwoven to form a coating on said target surface of the nonwoven;
**characterised in that**:
said aqueous solution comprises said hydrophilic polymer and said surfactant at a ratio of 30:70, whereby the hydrophilic polymer and surfactant coat said target surface on drying of the nonwoven;
the hydrophilic polymer and the surfactant are present in the aqueous solution at a total concentration ranging from 5% to 25% by weight of the total aqueous solution; and
the surface tension of said aqueous solution is less than 40 mN/m.

16. The method according to claim 15, wherein said nonwoven is employed as a topsheet in the absorbent article.

## Patentansprüche (Patentansprüche für folgende(n) Vertragsstaat(en): FR, DE, GB)

1. Absorbierender Gegenstand umfassend einen Vliesstoff aus hydrophoben Fasern, eine Rückschicht und einen Absorbenskern, der zwischen dem Vliesstoff und der Rückschicht angeordnet ist,
wobei wenigstens eine Zielfläche des Vliesstoffes mit einer wässrigen Lösung behandelt ist, die ein Tensid und ein hydrophiles Polymer umfasst, und wobei die wässrige Lösung getrocknet ist, um einen Überzug zu bilden;
**dadurch gekennzeichnet, daß**
der Überzug in einer Menge in einem Bereich von 0,10 bis 0,25 Gew.-% der Zielfläche vorliegt;
der Überzug 10 bis 50 Gew.-% an wasserlöslichem hydrophilem Polymer und 50 bis 90 Gew.-% des Tensids umfasst; und
der absorbierende Gegenstand ferner eine obere Schicht umfasst, wobei der Vliesstoff als eine Transferschicht dient, die zwischen dem Absorbenskern und der oberen Schicht positioniert ist.

2. Absorbierender Gegenstand nach Anspruch 1, wobei der Vliesstoff polyolefinische Fasern oder polyolefinischen Film umfasst.

3. Absorbierender Gegenstand nach einem der vorangehenden Ansprüche, wobei das hydrophile Polymer ausgewählt ist aus der Gruppe bestehend aus Aloe Vera, Methylcellulose, Hydroxypropylmethylcellulose, Gummiarabicum, Gummiacacia und Polyvinylalkohol.

4. Absorbierender Gegenstand nach Anspruch 3, wobei das hydrophile Polymer Aloe Vera ist.

5. Absorbierender Gegenstand nach einem der vorangehenden Ansprüche, wobei das Tensid ausgewählt ist aus einer Gruppe bestehend aus nicht-ionischen und anionischen Tensiden.

6. Absorbierender Gegenstand nach Anspruch 5, wobei das Tensid ausgewählt ist aus der Gruppe bestehend aus Dioctylnatriumsulfosuccinat (TRITON^{™}GR-5M) und Natriumalkylarylpolyethersulfonat (TRITON^{™}X-200).

7. Absorbierender Gegenstand nach Anspruch 5, wobei das Tensid ausgewählt ist aus einer Gruppe von Phospholipiden, die als Phosphoglyceride und Sphingolipide bekannt ist.

8. Absorbierender Gegenstand nach Anspruch 7, wobei das Tensid ausgewählt ist aus der Gruppe von Phosphoglyceriden bestehend aus Lecithinen und Cephalinen.

9. Absorbierender Gegenstand nach einem der vorangehenden Ansprüche, wobei das Tensid auf der Zielfläche in einer Menge von weniger als 0,125 Gew.-% der Zielfläche vorhanden ist.

10. Absorbierender Gegenstand nach einem der vorangehenden Ansprüche, wobei das hydrophile Polymer auf der Zielfläche in einer Menge im Bereich von 0,05 bis 1 Gew.-% der Zielfläche vorhanden ist.

11. Absorbierender Gegenstand nach Anspruch 10, wobei das hydrophile Polymer auf der Zielfläche in einer Menge von 0,05 Gew.-% der Zielfläche vorliegt.

12. Verfahren zum Behandeln eines Vliesstoffes zur Verwendung in einem absorbierenden Gegenstand, wie er in Anspruch 1 definiert ist, welches die Schritte umfasst:
Herstellen einer wässrigen Lösung umfassend ein Tensid und ein wasserlösliches hydrophiles Polymer;
Auftragen der wässrigen Lösung auf eine Zielfläche des Vliesstoffes; und
Trocknen des Vliesstoffes, um einen Überzug auf der Zielfläche des Vliesstoffes zu bilden;
**dadurch gekennzeichnet, daß**
die wässrige Lösung das hydrophile Polymer und das Tensid in einem Verhältnis von 30:70 umfasst, wobei das hydrophile Polymer und das Tensid die Zielfläche beim Trocknen des Vliesstoffes beschichten;
das hydrophile Polymer und das Tensid in der wässrigen Lösung in einer Gesamtkonzentration im Bereich von 5 bis 25 Gew.-% der gesamten wässrigen Lösung vorliegen; und
die Oberflächenspannung der wässrigen Lösung kleiner als 40 mN/m ist.

13. Verfahren nach Anspruch 12, wobei der Vliesstoff als eine obere Schicht in dem absorbierenden Gegenstand verwendet wird.

## Patentansprüche (Patentansprüche für folgende(n) Vertragsstaat(en): AT, BE, CH, CY, DK, ES, FI, GR, IE, IT, LI, LU, MC, NL, PT, SE)

1. Absorbierender Gegenstand, der einen Vliesstoff aus hydrophoben Fasern umfasst,
wobei wenigstens eine Zielfläche des Vliesstoffes mit einer wässrigen Lösung behandelt ist, die ein Tensid und ein hydrophiles Polymer umfasst, und wobei die wässrige Lösung getrocknet ist, um einen Überzug zu bilden;
**dadurch gekennzeichnet, daß**
der Überzug in einer Menge im Bereich von 0,10 bis 0,25 Gew.-% der Zielfläche vorliegt;
der Überzug 10 bis 50 Gew.-% des wasserlöslichen hydrophilen Polymers und 50 bis 90 Gew.-% des Tensids umfasst.

2. Absorbierender Gegenstand nach Anspruch 1, weiter umfassend eine obere Schicht, eine Rückschicht und einen Absorbenskern, der dazwischen angeordnet ist, wobei der Vliesstoff als eine Transferschicht dient, die zwischen dem Absorbenskern und der oberen Schicht positioniert ist.

3. Absorbierender Gegenstand nach Anspruch 1, wobei der Vliesstoff als eine obere Schicht des absorbierenden Gegenstands zum Kontakt mit der Haut eingesetzt wird.

4. Absorbierender Gegenstand nach Anspruch 2 oder 3, wobei der Vliesstoff polyolefinische Fasern oder polyolefinischen Film umfasst.

5. Absorbierender Gegenstand nach einem der vorangehenden Ansprüche, wobei das hydrophile Polymer ausgewählt ist aus der Gruppe bestehend aus Aloe Vera, Methylcellulose, Hydroxypropylmethylcellulose, Gummiarabicum, Gummiacacia und Polyvinylalkohol.

6. Absorbierender Gegenstand nach Anspruch 5, wobei das hydrophile Polymer Aloe Vera ist.

7. Absorbierender Gegenstand nach einem der vorangehenden Ansprüche, wobei das Tensid ausgewählt ist aus einer Gruppe bestehend aus nicht-ionischen und anionischen Tensiden.

8. Absorbierender Gegenstand nach Anspruch 7, wobei das Tensid ausgewählt ist aus der Gruppe bestehend aus Dioctylnatriumsulfosuccinat (TRITON^{™}GR-5M) und Natriumalkylarylpolyethersulfonat (TRITON^{™}X-200).

9. Absorbierender Gegenstand nach Anspruch 7, wobei das Tensid ausgewählt ist aus einer Gruppe von Phospholipiden, die als Phosphoglyceride und Sphingolipide bekannt ist.

10. Absorbierender Gegenstand nach Anspruch 9, wobei das Tensid ausgewählt ist aus der Gruppe der Phosphoglyceride bestehend aus Lecithinen und Cephalinen.

11. Absorbierender Gegenstand nach einem der vorangehenden Ansprüche, wobei das Tensid auf der Zielfläche in einer Menge von weniger als 0,125 Gew.-% der Zielfläche vorhanden ist.

12. Absorbierender Gegenstand nach einem der vorangehenden Ansprüche, wobei das hydrophile Polymer auf der Zielfläche in einer Menge im Bereich von 0,05 bis 1 Gew.-% der Zielfläche vorhanden ist.

13. Absorbierender Gegenstand nach Anspruch 12, wobei das hydrophile Polymer auf der Zielfläche in einer Menge von 0,05 Gew.-% der Zielfläche vorliegt.

14. Absorbierender Gegenstand nach Anspruch 3, wobei die obere Schicht eine Matrix von einzelnen Fasern umfasst und der Überzug auf den Fasern der Zielfläche angeordnet ist.

15. Verfahren zum Behandeln eines Vliesstoffes zur Verwendung in einem absorbierenden Gegenstand, wie er in Anspruch 1 definiert ist, welches die Schritt umfasst:
Herstellen einer wässrigen Lösung, die ein Tensid und ein wasserlösliches hydrophiles Polymer umfasst;
Auftragen der wässrigen Lösung auf eine Zielfläche des Vliesstoffes; und
Trocknen des Vliesstoffes, um einen Überzug auf der Zielfläche des Vliesstoffes zu bilden;
**dadurch gekennzeichnet, daß**:
die wässrige Lösung das hydrophile Polymer und das Tensid in einem Verhältnis von 30:70 umfasst, wobei das hydrophile Polymer und das Tensid die Zielfläche beim Trocknen des Vliesstoffes beschichten;
das hydrophile Polymer und das Tensid in der wässrigen Lösung in einer Gesamtkonzentration im Bereich von 5 bis 25 Gew.-% der gesamten wässrigen Lösung vorliegen; und
die Oberflächenspannung der wässrigen Lösung kleiner als 40 mN/m ist.

16. Verfahren nach Anspruch 15, wobei der Vliesstoff als eine obere Schicht in dem absorbierenden Gegenstand verwendet wird.

## Revendications (Revendications pour l'(les) Etat(s) contractant(s) suivant(s): FR, DE, GB)

1. Article absorbant comprenant un non-tissé de fibres hydrophobes, une feuille arrière ainsi qu'un noyau absorbant disposé entre ledit non-tissé et ladite feuille arrière, dans lequel au moins une surface cible dudit non-tissé est traitée avec une solution aqueuse comprenant un tensioactif et un polymère hydrophile, et ladite solution aqueuse est séchée pour former un revêtement ;
**caractérisé en ce que** :
- ledit revêtement est présent dans une quantité allant de 0,10 % à 0,25 % en poids de la surface cible ;
- ledit revêtement comprend de 10 % à 50 % en poids de polymère hydrophile hydrosoluble et de 50 % à 90 % en poids dudit tensioactif ; et
- ledit article absorbant comprend en outre une feuille supérieure, ledit non-tissé faisant office de couche de transfert positionnée entre ledit noyau absorbant et ladite feuille supérieure.

2. Article absorbant de la revendication 1, dans lequel ledit non-tissé comprend des fibres ou un film de polyoléfine.

3. Article absorbant de l'une quelconque des revendications précédentes, dans lequel ledit polymère hydrophile est choisi parmi le groupe constitué de l'aloe vera, la méthylcellulose, l'hydroxypropylméthylcellulose, la gomme arabique, la gomme d'acacia et l'alcool polyvinylique.

4. Article absorbant de la revendication 3, dans lequel ledit polymère hydrophile est de l'aloe vera.

5. Article absorbant de l'une quelconque des revendications précédentes, dans lequel le tensioactif est choisi parmi un groupe constitué de tensioactifs non ioniques et anioniques.

6. Article absorbant de la revendication 5, dans lequel le tensioactif est choisi parmi le groupe constitué du dioctylsulfosuccinate de sodium (TRITON^{™}GR-5M) et de l'alkylarylpolyéthersulfonate de sodium (TRITON^{™} X-200).

7. Article absorbant de la revendication 5, dans lequel le tensioactif est choisi parmi un groupe de phospholipides connus sous le nom de glycérophospholipides et de sphingolipides.

8. Article absorbant de la revendication 7, dans lequel ledit tensioactif est choisi parmi le groupe de glycérophospholipides constitué des lécithines et des céphalines.

9. Article absorbant de l'une quelconque des revendications précédentes, dans lequel ledit tensioactif est présent sur la surface cible dans une quantité inférieure à 0,125 % en poids de la surface cible.

10. Article absorbant de l'une quelconque des revendications précédentes, dans lequel ledit polymère hydrophile est présent sur la surface cible dans une quantité allant de 0,05 % à 1 % en poids de la surface cible.

11. Article absorbant de la revendication 10, dans lequel ledit polymère hydrophile est présent sur la surface cible dans une quantité de 0,05 % en poids de la surface cible.

12. Procédé pour traiter un non-tissé destiné à être utilisé dans un article absorbant tel que défini dans la revendication 1, comprenant les étapes consistant à :
- préparer une solution aqueuse comprenant un tensioactif et un polymère hydrophile hydrosoluble ;
- appliquer ladite solution aqueuse sur une surface cible du non-tissé ; et
- sécher le non-tissé pour former un revêtement sur ladite surface cible du non-tissé ;
**caractérisé en ce que** :
- ladite solution aqueuse comprend ledit polymère hydrophile et ledit tensioactif dans un rapport de 30:70, le polymère hydrophile et le tensioactif revêtant ladite surface cible après séchage du non-tissé ;
- le polymère hydrophile et le tensioactif sont présents dans la solution aqueuse à une concentration totale allant de 5 % à 25 % en poids de la solution aqueuse totale ; et
- la tension superficielle de ladite solution aqueuse est inférieure à 40 mN/m.

13. Procédé selon la revendication 12, dans lequel ledit non-tissé est employé en tant que feuille supérieure dans l'article absorbant.

## Revendications (Revendications pour l'(les) Etat(s) contractant(s) suivant(s): AT, BE, CH, CY, DK, ES, FI, GR, IE, IT, LI, LU, MC, NL, PT, SE)

1. Article absorbant comprenant un non-tissé de fibres hydrophobes, dans lequel au moins une surface cible dudit non-tissé est traitée avec une solution aqueuse comprenant un tensioactif et un polymère hydrophile, et ladite solution aqueuse est séchée pour former un revêtement ;
**caractérisé en ce que** :
- ledit revêtement est présent dans une quantité allant de 0,10 % à 0,25 % en poids de la surface cible ; et
- ledit revêtement comprend de 10 % à 50 % en poids de polymère hydrophile hydrosoluble et de 50 % à 90 % en poids dudit tensioactif.

2. Article absorbant de la revendication 1, comprenant en outre une feuille supérieure, une feuille arrière et un noyau absorbant disposé entre les deux, ledit non-tissé faisant office de couche de transfert positionnée entre ledit noyau absorbant et ladite feuille supérieure.

3. Article absorbant de la revendication 1, dans lequel ledit non-tissé est employé en tant que feuille supérieure de l'article absorbant pour entrer en contact avec la peau.

4. Article absorbant de la revendication 2 ou 3, dans lequel ledit non-tissé comprend des fibres ou un film de polyoléfine.

5. Article absorbant de l'une quelconque des revendications précédentes, dans lequel ledit polymère hydrophile est choisi parmi le groupe constitué de l'aloe vera, la méthylcellulose, l'hydroxypropylméthylcellulose, la gomme arabique, la gomme d'acacia et l'alcool polyvinylique.

6. Article absorbant de la revendication 5, dans lequel ledit polymère hydrophile est de l'aloe vera.

7. Article absorbant de l'une quelconque des revendications précédentes, dans lequel le tensioactif est choisi parmi un groupe constitué de tensioactifs non ioniques et anioniques.

8. Article absorbant de la revendication 7, dans lequel le tensioactif est choisi parmi le groupe constitué du dioctylsulfosuccinate de sodium (TRITON^{™}GR-5M) et de l'alkylarylpolyéthersulfonate de sodium (TRITON^{™} X-200).

9. Article absorbant de la revendication 7, dans lequel le tensioactif est choisi parmi un groupe de phospholipides connus sous le nom de glycérophospholipides et de sphingolipides.

10. Article absorbant de la revendication 9, dans lequel ledit tensioactif est choisi parmi le groupe de glycérophospholipides constitué des lécithines et des céphalines.

11. Article absorbant de l'une quelconque des revendications précédentes, dans lequel ledit tensioactif est présent sur la surface cible dans une quantité inférieure à 0,125 % en poids de la surface cible.

12. Article absorbant de l'une quelconque des revendications précédentes, dans lequel ledit polymère hydrophile est présent sur la surface cible dans une quantité allant de 0,05 % à 1 % en poids de la surface cible.

13. Article absorbant de la revendication 12, dans lequel ledit polymère hydrophile est présent sur la surface cible dans une quantité de 0,05 % en poids de la surface cible.

14. Article absorbant de la revendication 3, dans lequel ladite feuille supérieure comprend une matrice de fibres discontinues et le revêtement est déposé sur les fibres de la surface cible.

15. Procédé pour traiter un non-tissé destiné à être utilisé dans un article absorbant tel que défini dans la revendication 1, comprenant les étapes consistant à :
- préparer une solution aqueuse comprenant un tensioactif et un polymère hydrophile hydrosoluble ;
- appliquer ladite solution aqueuse sur une surface cible du non-tissé ; et
- sécher le non-tissé pour former un revêtement sur ladite surface cible du non-tissé ;
**caractérisé en ce que** :
- ladite solution aqueuse comprend ledit polymère hydrophile et ledit tensioactif dans un rapport de 30:70, le polymère hydrophile et le tensioactif revêtant ladite surface cible après séchage du non-tissé ;
- le polymère hydrophile et le tensioactif sont présents dans la solution aqueuse à une concentration totale allant de 5 % à 25 % en poids de la solution aqueuse totale ; et
- la tension superficielle de ladite solution aqueuse est inférieure à 40 mN/m.

16. Procédé selon la revendication 15, dans lequel ledit non-tissé est employé en tant que feuille supérieure dans l'article absorbant.
